# EUROPEAN PATENT SPECIFICATION

(11) **EP 3 007 715 B1**
(45) Date of publication and mention of the grant of the patent: **01.01.2020**
(21) Application number: 14730144.4
(22) Date of filing: 11.06.2014
(51) Int. Cl.: A61K 9/50, A61K 9/00, C12N 9/02, A61K 38/44, A61P 3/00

(54) **COMPOSITION OF ERYTHROCYTES ENCAPSULATING PHENYLALANINE HYDROXYLASE AND THERAPEUTIC USE THEREOF**
ZUSAMMENSETZUNG BASIEREND AUF ERYTHROCYTEN, IN DIE PHENYLALANIN-HYDROXYLASE VERKAPSELT IST, UND THERAPEUTISCHE VERWENDUNGEN DAVON.
COMPOSITION D'ÉRYTHROCYTES ENCAPSULANT PHENYLALANINE HYDROXYLASE ET L'EMPLOI THÉRAPEUTIQUE

(30) Priority: 11.06.2013 EP 13305786
(43) Date of publication of application: 20.04.2016
(73) Proprietor: Erytech Pharma, 69008 Lyon (FR)
(72) Inventor: GODFRIN, Yann, F-69004 Lyon (FR); DUFOUR, Emmanuelle, Lexington, MA 02421 (US); CHENG, Seng H., Natick, MA 01760 (US); YEW, Nelson S., Upton, MA 01568 (US)
(74) Representative: Lavoix
(86) International application number: PCT/EP2014/062156
(87) International publication number: WO 2014/198788

(56) References cited:
- EP-A1- 0 101 341
- WO-A1-96/39098

## Description

The present invention relates to Enzyme Replacement Therapy (ERT) based on phenylalanine hydroxylase and compositions intended for this use.

Elevated or altered levels of amino acids in blood are diagnostic for some inborn metabolic disorders. One example is phenylketonuria (PKU), an autosomal recessive disorder caused by a deficiency in phenylalanine hydroxylase (PAH), which metabolizes phenylalanine (Phe) to tyrosine in the presence of molecular oxygen, iron and the essential cofactor tetrahydrobiopterin (BH₄) [7, 8]. The enzyme is naturally found in the liver where it catalyzes the hydroxylation of L-phenylalanine to L-tyrosine using the co-factor BH₄ and molecular oxygen [22]. If untreated, Phe levels in the blood rise to extremely high levels, leading to neurocognitive decline and eventually severe mental retardation. Genetic screening now identifies newborns with PKU and they are immediately placed on a Phe-restricted diet [9]. However, compliance with the severe diet worsens during childhood and adolescence, and there remains a significant unmet medical need for this disorder [10].

ERT has often been hampered by the rapid clearance and degradation of the administered enzyme, limiting efficacy and requiring frequent dosing. The proteolytic sensitivity of proteic enzymes and potential immunogenicity of a recombinant enzyme or an enzyme from a microbial origin often preclude the use of unstabilized enzymes as a direct oral or injected therapy.

PKU is defined by the U.S. National Library of Medicine in the Genetics Home Reference (http://ghr.nlm.nih.gov/condition/phenylketonuria) as follows:
Phenylketonuria (commonly known as PKU) is an inherited disorder that increases the levels of a substance called phenylalanine in the blood. Phenylalanine is a building block of proteins (an amino acid) that is obtained through the diet. It is found in all proteins and in some artificial sweeteners. If PKU is not treated, phenylalanine can build up to harmful levels in the body, causing intellectual disability and other serious health problems.

The signs and symptoms of PKU vary from mild to severe. The most severe form of this disorder is known as classic PKU. Infants with classic PKU appear normal until they are a few months old. Without treatment, these children develop permanent intellectual disability. Seizures, delayed development, behavioral problems, and psychiatric disorders are also common. Untreated individuals may have a musty or mouse-like odor as a side effect of excess phenylalanine in the body. Children with classic PKU tend to have lighter skin and hair than unaffected family members and are also likely to have skin disorders such as eczema.

Less severe forms of this condition, sometimes called variant PKU and non-PKU hyperphenylalaninemia, have a smaller risk of brain damage. People with very mild cases may not require treatment with a low-phenylalanine diet.

Mammalian PAH is a large, multidomain, multimeric enzyme (200 kD as a tetramer) and, according to some authors, with relatively low specific activity [12]. The PAH from the cyanobacteria *Chromobacterium violaceum,* which is a 33 kD monomer is more thermally stable and reportedly more active than the mammalian enzyme [13]. Phenylalanine ammonia lyase (PAL) is a large (240 kD) tetrameric enzyme that converts Phe to trans-cinammic acid and ammonia.

WO96/39098 discloses a device intended to be implanted into a blood vessel and permitting a molecule to diffuse out the device into the blood stream. The molecule is secreted by viable cells present into a capsule in the device. Phenylalanine hydroxylase producing cells is proposed to treat phenylketonuria.

One who wishes to develop a PKU therapy is faced with the inherent protease sensitivity and potential immunogenicity of these enzymes. The cofactor requirement for the PAH is an additional difficulty. Protection of the enzyme against immune response and protease degradation has been achieved by polyethylene glycol (PEG) chemical conjugation (pegylation) of the protein. Full-length human PAH, double-truncated human PAH and *C. violaceum* PAH have been pegylated and displayed increased specific activity with respect to native form [22].

recites that the complex activity and cofactor requirement in addition to the inherent protease sensitivity and potential immunogenicity in a person lacking the functional enzyme makes the ERT a complicated one. [23] reminds that ERT with PAH requires the intact multienzyme complex for catalytic hydroxylating activity and that, to make it therapeutically viable, truncated forms of PAH have been constructed in an attempt to stabilize and increase catalytic activity while the protection of the enzyme against immune response has been achieved by pegylation of the protein. Nevertheless, [23] considers that given the drawbacks of the enzyme PAH, its viability as a therapeutic remains debatable. This is why [23] explored the PAL enzyme as a potential substitute for PAH and developed pegylated forms of this enzyme in order to overcome the potential proteolytic degradation and immunogenicity.

There is still a need for a novel and efficient ERT therapy for PKU.

Red blood cells (RBCs) have long been of interest as drug carriers and delivery vehicles, possessing several desirable features for this purpose, including complete biocompatibility, long lifespan (120 days in humans), and natural degradation and elimination by the body [1-4]. Purified populations of RBCs can be readily obtained in large quantities, and the methods for encapsulating small molecule drugs or proteins are straightforward [5]. As a delivery vehicle, encapsulated RBCs can be modified to be efficiently taken up by cells of the reticuloendothelial system (RES), delivering anti-inflammatory drugs, anti-cancer agents or antigens [1]. Alternatively, enzyme-encapsulated RBCs can serve as circulating bioreactors that can continuously detoxify toxins or excess metabolites present in the blood [4]. In this regard, channels and transporters present in the erythrocyte membrane allow the transport of ions, amino acids, and various small molecules into and out of the cell [6]. Importantly, encapsulated proteins considered foreign by the immune system are shielded from any neutralizing antibodies present in the circulation. The encapsulation process involves reversible hypotonic swelling of the RBCs that transiently opens pores in the membrane, allowing drugs to enter the cells [5, 11]. Upon return to isotonic medium the pores are closed and the drug is entrapped. While the procedure is highly reproducible, the inherent properties of the encapsulated protein are critical, with efficacy dependent on the stability and specific activity of the enzyme.

Given that the basic concept of encapsulating enzymes within RBCs (e.g. EP 101 341) has been in existence for a while, the number of examples and applications using this approach over the past several years is relatively few [16]. One of the most promising and advanced applications has been to encapsulate asparaginase for the treatment of acute lymphoblastic leukemia (ALL) [17, 18]. While asparaginase is widely used to treat ALL, the enzyme has a short half-life in plasma (from 15 to 30 h depending on the bacterial source of the protein), thus requiring frequent re-administration and inducing significant adverse immune responses and general toxicity. Previous and ongoing clinical trials using asparaginase-loaded RBCs have shown greatly improved pharmacokinetics and pharmacodynamics compared to the free enzyme and significantly reduced adverse immune effects [4, 19-21].

The erythrocyte bioreactor model may function if several prerequisites are simultaneously present, in particular the enzyme is stable and active within the erythrocytes, the erythrocytes are not degraded by the enzyme, and the substrate for the enzyme enters at a sufficient rate into the RBCs. The situation is much more complicated in the case of PAH which further requires its cofactor BH₄.

In line with the recent developments on PAL as replacement enzyme, the present applicant has encapsulated PAL into RBCs and observed that PAL-loaded RBCs are able to lower Phe levels in normal mice (data not shown). However, the pharmacokinetics of PAL-RBCs were uncharacteristically short, with PAL being lost from the circulation within 24 hours. This may be explained by the very high sensitivity of PAL to degradation, which drawback is also present with PAH.

To begin to develop therapies for PKU, the applicant has however evaluated the use of RBC encapsulated with PAH in mice. Unexpectedly, a prokaryotic, monomeric form of PAH was entrapped into mouse RBCs and demonstrated to function as phenylalanine metabolizing bioreactors when injected into the bloodstream, the PAH-RBCs were able to take up both Phe and the necessary cofactor BH₄ from the circulation and convert phenylalanine to tyrosine, and, compared to free PAH protein, which survived in the blood for less than one hour, and PAL-RBCs, the PAH-RBCs persisted in the circulation and remained active for at least 10 days.

Thus the present invention concerns in particular:
- an erythrocyte (or RBC) encapsulating PAH, especially in suspension in a pharmaceutically acceptable carrier or vehicle;
- a pharmaceutical composition comprising erythrocytes encapsulating PAH in a pharmaceutically acceptable carrier or vehicle;
- a pharmaceutical composition comprising erythrocytes encapsulating PAH in a pharmaceutically acceptable carrier or vehicle, for use in the treatment or prevention of phenylketonuria (PKU) and/or other diseases involving a too high level of phenylalanine; the treatment or prevention may be in combination with a Phe-restricted diet;
- a method of treatment or prevention of phenylketonuria (PKU) and/or other diseases involving a too high level of phenylalanine, comprising the administration to a patient in need thereof of an effective amount of a pharmaceutical composition comprising erythrocytes encapsulating PAH in a pharmaceutically acceptable carrier or vehicle; the treatment or prevention may be in combination with a Phe-restricted diet.

Definition of the PKU and the different grades of the disorder is the one given by the U.S. National Library of Medicine in the Genetics Home Reference (http://ghr.nlm.nih.gov/condition/phenylketonuria).

In an embodiment of the invention, classic PKU is concerned. Thus the invention concerns this pharmaceutical composition for use in the treatment or prevention of classic PKU, or the method of treatment or prevention to the benefit of a patient having or against classic PKU.

In an embodiment, variant PKU and/or non-PKU hyperphenylalaninemia is/are concerned. Thus the invention concerns this pharmaceutical composition for use in the treatment or prevention of variant PKU and/or non-PKU hyperphenylalaninemia, or the method of treatment or prevention to the benefit of a patient having or against variant PKU and/or non-PKU hyperphenylalaninemia.

Phe-restricted diet is well known to the person skilled in the art. It is known as low-phenylalanine diet, which means that the diet comprises providing meals having no or a low content of Phe.

By PAH it is intended especially full-length human PAH, a modified human PAH, such as the double-truncated human PAH [22], a prokaryotic, monomeric form of PAH, such as *C*. *violaceum* PAH, or any other full-length or modified PAH which is capable of catalyzing the hydroxylation of L-phenylalanine to L-tyrosine using the co-factor BH₄ and molecular oxygen. Each one of these recited PAH also constitutes an embodiment of the invention.

Typically, the erythrocytes are in suspension in a pharmaceutically acceptable saline solution. This can be a standard medium for erythrocytes, in particular a solution of NaCl (preferably about 0.9%) possibly with added ingredients such as glucose, dextrose, adenine and/or mannitol.

In an embodiment, the solution comprises NaCl, adenine and dextrose. For example, the AS3 medium is used.

In another embodiment, the solution comprises NaCl, adenine, glucose and mannitol. Standard media that can be used are SAG mannitol and ADsol.

The solution can further contain a preservative such as L-carnitine.

In an embodiment, one dose of suspension comprises from 50 to 2000 IU, preferably from 100 to 1000 IU of encapsulated PAH. By definition, a dose is the amount of PAH administered to the patient at a given time.

"Encapsulated" means that the enzyme is contained inside the erythrocytes. It is possible however that some minor amount of PAH is retained within the erythrocyte wall.

The composition may be ready for use and have a haematocrit suitable for administration by injection or by perfusion without dilution.

In an embodiment, the composition is ready for use. The haematocrit of the suspension ready for use advantageously lies between about 40 and about 70%, preferably between about 45 and about 55%, and better about 50%.

In another embodiment, the composition has to be diluted before use, e.g. before administration by injection or by perfusion. In an embodiment of such a composition to be diluted before use, the haematocrit before dilution lies between 60 and 90%, and after dilution, the ready to use composition is as mentioned above.

The composition is preferably packaged at a volume of about 10 to about 250 ml. The packaging is preferably in a blood bag of the type suitable for a blood transfusion. The whole of the quantity of encapsulated PAH corresponding to the medical prescription is preferably contained in one blood bag and the like. It may also be contained in several blood bags and the like.

Techniques for encapsulating active principle in red blood cells are known and the basic technique by lysis-resealing, which is preferred herein, is described in patents EP-A-101 341 and EP-A-679 101, to which those skilled in the art may refer. According to this technique, the primary compartment of a dialysis element (for example, a dialysis tubing or a dialysis cartridge) is continuously fed with a suspension of red blood cells, while the secondary compartment contains an aqueous solution which is hypotonic with respect to the suspension of red blood cells, in order to effect lysis (reversible lysis) of the red blood cells; next, in a resealing unit, the resealing of the red blood cells is induced in the presence of PAH by increasing the osmotic and/or oncotic pressure, and then a suspension of red blood cells containing PAH is collected.

Among the variants described up until now, preference is given to the method described in WO2006/016247, which makes it possible to efficiently, reproducibly, safely and stably encapsulate PAH. This method comprises the following steps:
1 - suspension of a red blood cell pellet in an isotonic solution at a haematocrit level greater than or equal to 60 or 65%, cooling between + 1 and + 8°C,
2 - measurement of the osmotic fragility using a sample of red blood cells from said red blood cell pellet,
   it being possible for steps 1 and 2 to be carried out in any order (including in parallel); it is preferable to prepare the suspension in 1-, and to measure the osmotic fragility on a sample of this suspension;
3 - lysis and internalization process of PAH, at a temperature constantly maintained between + 1 and + 8°C, comprising passing the suspension of red blood cells at a haematocrit level greater than or equal to 60 or 65%, and a hypotonic lysis solution cooled to between + 1 and 8°C, through a dialysis cartridge; and the lys is parameters being adjusted according to the osmotic fragility previously measured; preferably, the flow rate of erythrocyte suspension or the osmolarity of the lysis solution is adjusted; and
4 - resealing process carried at a temperature between + 30 and + 40°C, and preferably in the presence of a hypertonic solution.

The "internalization" is intended to mean penetration of PAH inside the red blood cells.

In particular, for the dialysis, the red blood cell pellet is suspended in an isotonic solution at a high haematocrit level, greater than or equal to 60 or 65%, and preferably greater than or equal to 70%, and this suspension is cooled to between + 1 and + 8°C, preferably between + 2 and 6°C, typically at about + 4°C. According to a specific embodiment, the haematocrit level is between 65% and 80%, preferably between 70% and 80%.

The osmotic fragility is advantageously measured on the red blood cells just before the lysis step. The red blood cells or the suspension containing them are (is) advantageously at a temperature close to or identical to the temperature selected for the lysis. According to another advantageous feature of the invention, the osmotic fragility measurement is exploited rapidly, i.e. the lysis process is carried out shortly after the sample has been taken. Preferably, this period of time between taking the sample and beginning the lysis is less than or equal to 30 minutes, more preferably still less than or equal to 25, and even less than or equal to 20 minutes.

As regards the manner in which the lysis-resealing process is carried out, with the osmotic fragility being measured and taken into account, those skilled in the art may refer to WO2006/016247 for further details.

According to one feature of the invention, the composition according to the invention comprises, at the end of the lysis-resealing process, a suspension of red blood cells at a haematocrit level of between about 40% and about 70%, preferably between about 45% and about 55%, better still about 50%. It is preferably packaged in a volume of about 10 to about 250 ml. The packaging is preferably in a blood bag, syringe and the like, of a type suitable for blood transfusion or administration. The haematocrit may be adjusted to provide for a ready-to-use composition or a to-be extemporaneously diluted composition. The amount of encapsulated PAH corresponding to the medical prescription is preferably entirely contained in the blood bag, syringe and the like.

Cofactor BH₄ may be added to the pharmaceutical composition. The cofactor may be added before or shortly before administration of the composition.

The pharmaceutical composition according to the invention comprising erythrocytes encapsulating PAH in a pharmaceutically acceptable carrier or vehicle, as disclosed herein, is for use in the treatment or prevention of phenylketonuria and/or other diseases involving a too high level of phenylalanine, in particular of PKU.

In an embodiment, the composition is for use in such treatment or prevention in a patient in combination with a Phe-restricted diet. Preferably, the patient is already under a Phe-restricted diet.

In another embodiment, the composition is for use in the treatment or prevention on a patient having a classic form of PKU or in risk of developing a classic form of PKU.

In another embodiment, the composition is for use in the treatment or prevention in a patient in combination with a Phe-restricted diet and the patient has a classic form of PKU or is in risk of developing a classic form of PKU. Preferably, the patient is already under a Phe-restricted diet.

In another embodiment, the composition is for use in the treatment or prevention in a patient having a variant PKU and/or non-PKU hyperphenylalaninemia or in risk of developing a variant PKU and/or non-PKU hyperphenylalaninemia.

In still another embodiment, the composition is for use in the treatment or prevention in a patient with a Phe-restricted diet and the patient has a variant PKU and/or non-PKU hyperphenylalaninemia or is in risk of developing a variant PKU and/or non-PKU hyperphenylalaninemia. Preferably, the patient is already under a Phe-restricted diet.

According to a feature, the composition is for use in combination with, or in a patient receiving doses, of the cofactor BH₄. Modalities for the associated cofactor treatment are disclosed herein with respect to the method of treatment.

The method of treatment or prevention of phenylketonuria and/or other diseases involving a too high level of phenylalanine, in particular PKU, comprises the administration to a patient in need thereof of an effective amount of a pharmaceutical composition comprising erythrocytes encapsulating PAH in a pharmaceutically acceptable carrier or vehicle, as disclosed herein.

In an embodiment, in this method, the treatment or prevention is combined with a Phe-restricted diet. Preferably, the administration of the composition according to the invention is performed in a patient who is already under a Phe-restricted diet.

In another embodiment, the treatment or prevention is applied to a patient having a classic form of PKU or who is in risk of developing a classic form of PKU

In another embodiment, the treatment or prevention is applied to a patient having a variant PKU and/or non-PKU hyperphenylalaninemia or who is in risk of developing a variant PKU and/or non-PKU hyperphenylalaninemia.

In another embodiment, the treatment or prevention is combined with a Phe-restricted diet and the patient has a classic form of PKU or is in risk of developing a classic form of PKU. Preferably, the administration of the composition according to the invention is performed in a patient who is already under a Phe-restricted diet.

In still another embodiment, the treatment or prevention is combined with a Phe-restricted diet and the patient has a variant PKU and/or non-PKU hyperphenylalaninemia or is in risk of developing a variant PKU and/or non-PKU hyperphenylalaninemia. Preferably, the administration of the composition according to the invention is performed in a patient who is already under a Phe-restricted diet.

Administration is preferably effected by intravenous or intra-arterial injection. In a convenient embodiment, administration is performed by perfusion from a blood bag or the like. Administration is typically effected intravenously into the arm or via a central catheter. Typically one dose is perfused or infused and this may last from about 15 to 45 minutes.

According to one feature of the invention, about 10 to about 250 ml, typically about 10 to about 200 ml of a suspension of red blood cells is administered. The suspension is at an appropriate haematocrit level, generally between about 40% and about 70%, preferably between about 45% and about 55%, better still about 50%, are administered.

According to a feature, an efficient amount of the cofactor BH₄ is administered to the patient. Typically, the cofactor is administered daily. The administration of the cofactor may begin before, simultaneously or after the administration of the composition of the invention. The cofactor may be administered at least over a period during which the patient is under treatment with the composition of the invention. The patient may be administered with the cofactor daily over the whole life.

The cofactor may also be added to the composition before administration to the patient. Therefore, the composition according to the invention may comprise the cofactor.

The invention will now be described in greater detail using examples taken as embodiments and in reference to the Figures.

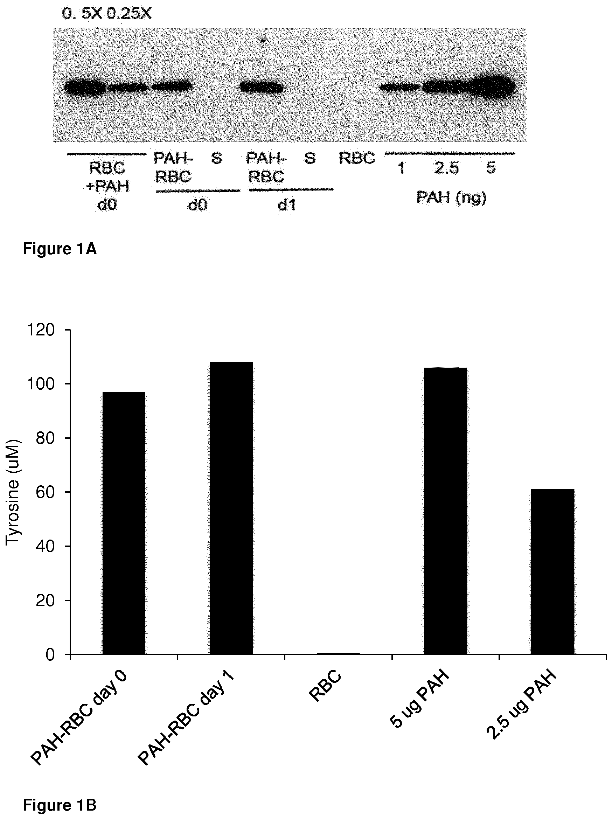

**Figure 1****.** Encapsulation of *C.violaceum* PAH in RBCs. A) Western blot to estimate the amount of PAH entrapped within the RBCs. The mixture of RBCs and PAH prior to encapsulation (RBC+PAH), day 0 (d0) and day 1 (d1) after encapsulation (PAH-RBC), the supernatant of the PAH-RBC suspension (S), or RBCs alone (RBC). An equivalent of 5 nL of each sample was loaded per lane, with the exception that the RBC+PAH sample was diluted 0.5X and 0.25X before loading (i.e. an equivalent of 2.5 and 1.25 nL of sample loaded). The blot was probed with an anti-PAH antibody. B) Enzyme activity of the PAH-RBCs. 10 µL of the PAH-RBCs was assayed on day 0 and day 1.

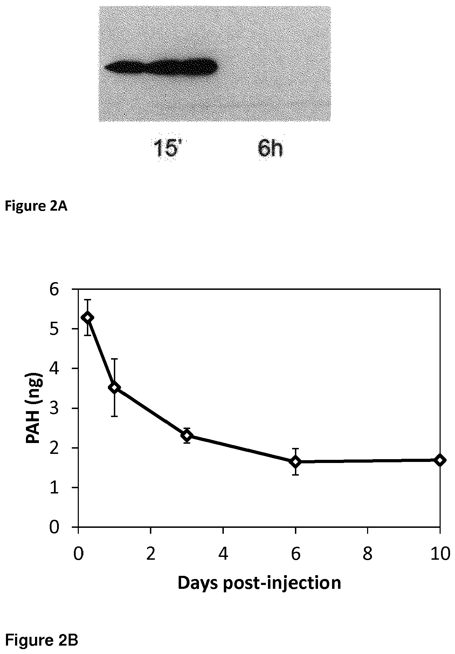

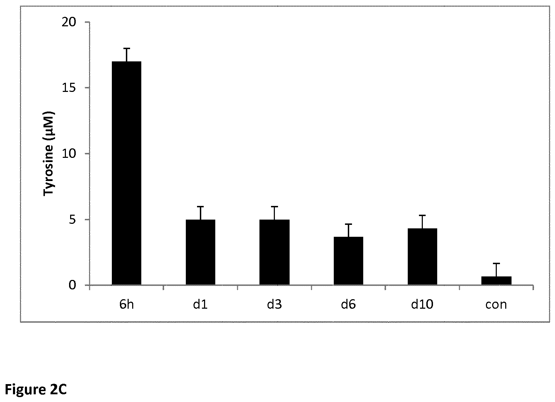

**Figure 2****.** Pharmacokinetics of free PAH and PAH-RBCs after IV administration into normal mice. A). Western blot of whole blood 15' and 6 h after injection of free PAH enzyme (4 mg/kg, i.e. 0.1 mg for a 25 g mouse). An equivalent of 0.5 µL of blood was loaded per lane. N = 3 mice per time point. B) Quantitation of PAH levels in blood over time after injection of PAH-RBCs. A Western blot of washed RBCs from blood samples taken at 6 h, 1, 3, 6, and 10 days post-injection was performed and the PAH signal quantitated by densitometry. Signal was compared to purified PAH standards. C) PAH enzyme activity in blood over time after injection of PAH-RBCs. PAH activity was measured from whole blood samples taken at 6 h, 1, 3, 6, and 10 days post-injection. N = 3 mice per time point. The data are expressed as the mean +/- SEM.

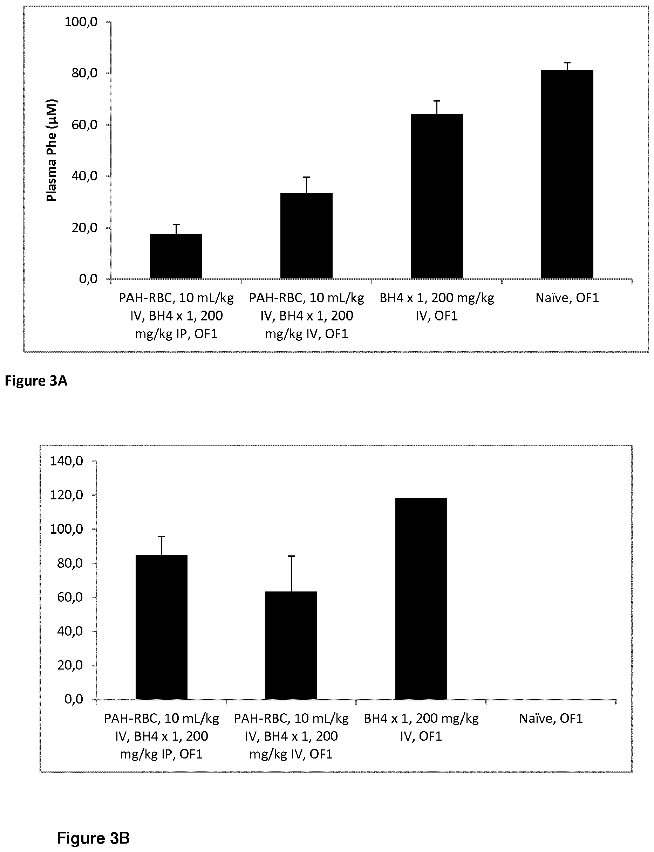

**Figure 3****.** Effect of IV administration of PAH-RBCs on plasma Phe levels 6 h post-injection of PAH-RBCs into normal mice. BH₄ was injected IP or IV (200 mg/kg) 5.5 h after injection of PAH-RBCs. N = 3 mice per group. B) Plasma BH₄ levels 6 h post-injection of PAH-RBCs. N= 1 for IV alone group. Het, heterozygous mice. N = 8 mice per group (N = 3 mice Naïve het). **P < 0.001, *P < 0.01. The data are expressed as the mean +/- SEM.

### Examples:

### Expression and purification of recombinant C. violaceum PAH

The sequence encoding *C. violaceum* PAH (GenBank accession #AF6711) was codon optimized for expression in *E. coli* and synthesized by DNA2.0 (Menlo Park, CA USA). The synthetic sequence was cloned into the Ndel and Xhol sites of pET29a(+) (EMD Millipore, Darmstadt, Germany) so that the 6XHis tag sequence was added in-frame to the 3' end of the PAH gene. The pET29a-PAH construct was used to transform BL21 (DE3) star cells (Life Technologies, Carlsbad, CA USA), which were induced with 0.5 mM IPTG at 20°C for 18 h. Cell pellets were resuspended in lysis buffer (50 mM Tris-HCI, pH 7.4, 150 mM NaCl, 5 mM β-mercaptoethanol, protease inhibitors). The cell suspension was sonicated then centrifuged at 17,000 x g for 30 min. The supernatant was decanted and FeSO₄ was added to a final concentration of 0.6 mM. The supernatant was stirred for 30 min at 4°C then filtered through a 0.2 µm filter.

A 5 mL HisTrap FF column (GE Healthcare Life Sciences, Pittsburgh, PA USA) was equilibrated with 10 column volumes (CV) of equilibration buffer (50 mM Tris-HCI, pH 7.4, 150 mM NaCl, 5 mM β-mercaptoethanol, 5 mM imidazole). After loading the supernatant, the column was washed with 50 CV of Triton X-114 buffer (50 mM Tris-HCI, pH 7.4, 150 mM NaCl, 5mM β-mercaptoethanol, 0.1% Triton X-114) followed by 20 CV of equilibration buffer. The protein was then eluted with elution buffer (50 mM Tris-HCI, pH 7.4, 150 mM NaCl, 5 mM β-mercaptoethanol, 300 mM imidazole). The protein containing fractions were pooled, buffer-exchanged into 50 mM sodium acetate, pH 6.2, concentrated by ultrafiltration (Amicon PD-10 columns) (EMD Millipore) and stored at -20°C.

### PAH enzyme assay

The activity of purified *C. violaceum* PAH was measured according to the procedure described by Nakata et al. [13] with some modifications. The assay mixture contained 100 mM Tris-HCI, pH 7.5, 4 mM DTT, 4 mM L-phenylalanine, 100 µg bovine catalase, and 10-50 µg of PAH enzyme in a volume of 250 µL. The reaction was initiated by the addition of DMPH₄ to a final concentration of 0.4 mM and was carried out at 23°C for 0.5-1 h with shaking. For PAH activity measurements of blood samples, 10-20 µL of washed RBCs was added to the mixture and the reaction time increased to 2-6 h. The reaction was stopped by the addition of 250 µL of 5% TCA. Tyrosine was detected colorimetrically by adding 250 µL of 0.1% 1-nitroso-2-napthol in 100 mM NaOH and 250 µL of 20% (v/v) HNO₃ with 0.05% (w/v) NaNO₂. The mixture was incubated at 55°C for 30 min, cooled to 25°C, then centrifuged 3 min x 5,000 rpm. The supernatant was transferred to a microplate and read at 450 nM. Values were compared to tyrosine standards (0-280 µM).

### Encapsulation of PAH in RBCs

The PAH enzyme was loaded into mouse RBCs by the method of reversible hypotonic dialysis using an AN69 hollow fiber dialyzer (Gambro, Lyon, France) as described by Dufour *et al.* [24] with some modifications. Whole blood from OF1 mice was centrifuged and the plasma was removed. The RBCs were washed three times with 0.9% (v/v) NaCl. Purified PAH was then added to the washed RBCs to a final concentration of 1 mg/mL, resulting in a cell suspension with a hematocrit of approximately 70%. The RBCs plus PAH were dialyzed against a hypotonic buffer of 40 mOsmol/kg and then resealed with the addition of a hypertonic solution (10% v/v) as described previously [25]. The suspension was then incubated at 37°C for 30 min. After final washes, the final product was resuspended with SAG-Mannitol plus 6% bovine serum albumin to a hematocrit of 50%. The product was stored at 2-8°C and was injected no more than 16 hours after preparation.

As a control, processed control RBCs (CON-RBCs) were prepared by dialyzing RBCs as described above but without added PAH.

### Estimation of PAH levels in PAH-RBCs and in blood

A 5 µL aliquot of the PAH-RBCs was serially diluted with phosphate-buffered saline (PBS) such that an equivalent of 5 nL of the PAH-RBCs was loaded onto a 4-15% acrylamide gel (Bio-Rad, Hercules, CA). After electrophoresis and transfer to nitrocellulose, the blot was probed with a rabbit polyclonal antibody to PAH (1:10,000 dilution of a 1 mg/mL stock). The antibody was generated by immunizing rabbits with the purified *C*. *violaceum* PAH protein. A goat anti-rabbit secondary antibody (Santa Cruz Biotechnology, Dallas, TX) followed by a chemiluminescent substrate (SuperSignal West Pico chemiluminescent substrate, Thermo Scientific, Rockford, IL) was used to detect the bands.

For detecting levels of PAH in the circulation, whole blood was centrifuged to pellet the RBCs, which were washed 2X with PBS. An equivalent of 0.5 µL of washed RBCs was loaded per lane.

### Mice

OF1 mice, 6-8 weeks old, were purchased from Charles River Laboratories (Lyon, France). PAH^{enu2} mice were purchased from Jackson Laboratories (Bar Harbor, ME, USA) and a colony was maintained in-house. The PAH^{enu2} animals were cared for in an AAALAC accredited facility in accordance with the guidelines established by the National Research Council. Animals had access to food and water *ad libitum.*

### In Vivo Studies

The PAH-RBCs or negative control processed RBCs were injected intravenously (10-12 mL/kg) via the tail vein. Tetrahydrobiopterin was injected intraperitoneally (200 mg/kg) 30 min prior to bleeding the animals. Blood was collected under anesthesia (3-5% isoflurane) via the orbital venous plexus using microcapillary tubes. An aliquot of whole blood was frozen at -80°C for enzyme activity analysis. The remaining blood was centrifuged, plasma was collected and frozen at -80°C. The pelleted RBCs were washed 2X with phosphate-buffered saline (PBS), then resuspended to 50% hematocrit in PBS and frozen at -80°C.

### Measurement of Phenylalanine and BH₄ Levels in Plasma

The levels of Phe and BH₄ in plasma were analyzed by UPLC-MS/MS, using an Acquity UPLC (Waters Corporation, Milford, MA, USA) hyphenated to an API 5000 triple quadropole mass spectrometer (AB SCIEX, Framingham, MA, USA). L-phenylalanine (Sigma-Aldrich, St. Louis, MO, USA) was used to prepare standard solutions, and labeled L-phenylalanine-¹³C, ¹⁵N (Sigma-Aldrich) was used as the internal standard. Analysis of Phe was performed using an Acquity BEH C18 column (1.7 mm, 2.1 mm x 50 mm) with gradient separation, which included a 0.5 min hold at 100% (0.5% trifluoroacetic acid, 0.3% heptafluorbutyric acid in water) followed by a 0-30% acetonitrile gradient. MS/MS transitions were: 166.1/120.1 for phenylalanine and 176.1/129.1 for labeled phenylalanine.

BH₄ is highly unstable, therefore 100 ng/mL cysteine, 0.1% dithioerythritol was used as the sample diluent buffer to minimize oxidation. Analysis of BH₄ was performed using a Phenomenex Luna 3u C18(2) Mercury 2.0 x 2.0 column cartridge kept at 30°C. The chromatographic run was performed at 1 mL/min flow under isocratic conditions with a mobile phase consisting of 1% acetonitrile with 99% of a 0.5% trifluoroacetic acid, 0.3% heptafluorobutyric acid solution in water. The overall run time was 1 min with a 1 µL injection volume. The electrospray ionization (ESI) was carried out in the positive-ion mode. Multiple reaction monitoring (MRM) mode of the precursor-product ion transition was 242.0/166.1. The declustering potential (DP) and collision energy (CE) manually optimized to 50 and 25, respectively, while the ion-spray potential and temperature were set to 1,500 V and 500 °C.

### Statistical Analysis

Data were analyzed by one-way analysis of variance (ANOVA) followed by Bonferroni's post-test. Statistical significance was assigned as follows: * indicates P < 0.01 and ** indicates P<0.001.

### RESULTS

### Purification of recombinant C. violaceum PAH from E. coli

The sequence encoding PAH from *C. violaceum* was codon-optimized for expression in *E. coli,* synthesized, and then the synthetic gene was cloned into the inducible plasmid expression vector pET29a such that a 6X His tag was incorporated into the C-terminus of the protein. Expression of PAH was induced by IPTG, and the protein was purified from the soluble *E. coli* lysate by affinity chromatography over a nickel-agarose resin. The column was washed extensively with 0.1% Triton-X114 buffer to remove endotoxin. Approximately 130 mg of protein of >90% purity was obtained from six liters of culture. Endotoxin levels were typically less than 0.5 EU/mg and the specific activity of the purified enzyme was -0.3 U/mg.

### Stability of purified PAH in vitro

A prerequisite for encapsulating an enzyme within RBCs is that the enzyme should be stable and not prone to inactivation. The stability of solutions of purified PAH were evaluated *in vitro* by incubation at 4, 23, or 37°C for 21 days. The enzyme exhibited little loss of activity over the first 14 days at all the temperatures tested, with a moderate decrease in activity at 37°C only after three weeks of incubation. Thus, the results indicated that the purified PAH was quite stable over time *in vitro.*

### Encapsulation of C. violaceum PAH into mouse RBCs

We next encapsulated the purified PAH protein into normal mouse RBCs by reversible hypotonic dialysis and resealing. The procedure utilizes a hollow fiber dialyzer to transiently disrupt the erythocyte membrane under controlled conditions such that hemolysis is minimal, and it is currently the only procedure that can process a sufficient volume of blood for clinical use. The hematologic characteristics of a representative batch are shown in Table 1. The corpuscular hemoglobin concentration decreased only slightly compared to the concentration before dialysis, indicating that hemoglobin was largely retained within the PAH-loaded RBCs. The low extracellular hemoglobin concentration indicates that there was little hemolysis of the PAH-RBCs upon storage overnight at 4°C. There was only a gradual decline in cell integrity with longer storage of up to seven days (data not shown).

**Table 1. Hematologic characterization of PAH-RBCs**

| | Day 0 | | Day 1 |
|---|---|---|---|
| | RBC + PAH | PAH - RBC | PAH - RBC |
| Hematocrit (%) | 73.7 | 49.4 | 47.3 |
| Globular volume (µm³) | 54.4 | 47.8 | 46.6 |
| Extracellular hemoglobin (g/dL) | NM* | 0.2 | 1.0 |
| Corpuscular hemoglobin (g/dL) | 27.7 | 23.4 | 23.5 |
| RBC/mL (X10⁹) | 15.8 | 12.9 | 13.2 |
| *NM : Not measured | | | |

To determine the efficiency of encapsulation and amount encapsulated, the mixture of RBCs and purified PAH before encapsulation as well as the final encapsulated product were analyzed by immunoblotting. The RBC plus PAH mixture before encapsulation was diluted 2- and 4-fold prior to loading the sample on a gel to prevent oversaturation of the signal. By comparison to purified PAH standards, approximately one-quarter of the initial PAH was successfully encapsulated within the RBCs, with the final product containing approximately 0.4 mg of PAH per mL (Fig. 1A). Analysis of the PAH-RBCs after one day of storage at 4°C showed that the cells remained intact with no detectable leakage of PAH into the supernatant. Enzymatic analysis of the PAH-RBCs showed that the PAH enzyme remained active after encapsulation, with no loss of activity after one day of storage at 4°C (Fig. 1B).

### Pharmacokinetics of PAH-RBCs in normal mice

To determine the pharmacokinetics of the encapsulated PAH *in vivo,* mice were injected intravenously with PAH-RBCs at a dose of 10 mL/kg. An equivalent amount of free PAH was injected into a separate group of mice for comparison, and blood was collected at 15', 6 h, 1, 3, 6, and 10 days post-injection. Although abundant levels of PAH were present at the 15' timepoint in the group injected with free PAH, circulating enzyme was undetectable by 6 h post-injection (Fig. 2A). In contrast, PAH levels in the blood of mice injected with the PAH-RBCs declined over the first few days but persisted through day 10 (Fig. 2B). Enzyme activity of washed RBCs sampled at each time point showed that PAH activity was present for the duration of the study (Fig. 2C). The results demonstrate that encapsulation of PAH within RBCs greatly improved the pharmacokinetics of the enzyme in the circulation.

### Efficacy of PAH-RBCs in normal mice

We next asked if the injected PAH-RBCs were capable of metabolizing Phe in the blood. Mice were injected with PAH-RBCs (10 mL/kg) and Phe levels were measured 6 h post-injection. Since PAH requires the cofactor BH₄ for enzymatic activity, BH₄ was injected either intravenously or intraperitoneally 30 min prior to bleeding the animals. The time of injection was chosen based on the reported short half-life and rapid clearance of BH₄ *in vivo* [14, 15]. Injection of BH₄ alone resulted in a small decrease in Phe levels, possibly due to activation of the endogenous liver PAH present in these normal animals (Fig. 3A). High levels of BH₄ were present in the blood at the 6 h time point after either IV or IP injection of BH₄ at 5.5 h after injection of the PAH-RBCs (Fig. 3B). Administration of the PAH-RBCs along with either IV or IP injection of BH₄ resulted in a substantial decrease in Phe levels, 59 and 78% respectively (P < 0.001) (Fig. 3A). There was a corresponding increase in plasma tyrosine levels in the groups injected with the PAH-RBCs (data not shown). The results demonstrated that the PAH-RBCs could effectively metabolize Phe to tyrosine in the blood and lower Phe levels in normal mice.

### Encapsulation of PAH in human erythrocytes

The method described in WO-A-2006/016247 is used to produce a batch of erythrocytes encapsulating PAH. In accordance with the teaching of WO-A-2006/016247, the osmotic fragility is considered and the lysis parameters are adjusted accordingly (flow rate of the erythrocyte suspension in the dialysis cartridge is adjusted). The method is further performed in conformity with the physician prescription, which takes into account the weight of the patient and the dose of PAH to be administered. The specifications of the end product are as follows:
- mean corpuscular volume (MCV): 70-95 fL
- mean corpuscular haemoglobin concentration (MCHC): 23-35 g/dL
- extracellular haemoglobin ≤ 0.2 g/dL of suspension
- osmotic fragility ≤ 6 g/L of NaCl
- extracellular PAH ≤ 2 % of the total enzyme activity.

The erythrocytes according to the invention may be defined by one or several of these specifications, in particular by the mean corpuscular haemoglobin concentration (MCHC) of 23-35 g/dL.

### REFERENCES

1. Muzykantov, VR (2010). Expert Opin Drug Deliv 7: 403-427.
2. Magnani, M, Pierige, F, and Rossi, L (2012). Ther Deliv 3: 405-414.
3. Godfrin, Y, et al. (2012). Expert Opin Biol Ther 12: 127-133.
4. Godfrin, Y, and Bax, BE (2012). Drugs of the Future 37: 263-272.
5. Hamidi, M, and Tajerzadeh, H (2003). Drug Deliv 10: 9-20.
6. Tunnicliff, G (1994).. Comp Biochem Physiol Comp Physiol 108: 471-478.
7. Scriver, CR, and Kaufman, S (2001). Hyperphenylalanemia: phenylalanine hydroxylase deficiency. In: Scriver, CR, AL Beaudet, WS Sly and D Valle eds). The Metabolic and Molecular Bases of Inherited Disease, 8th ed. McGraw-Hill: New York. pp 1667-1724.
8. Blau, N, van Spronsen, FJ, and Levy, HL (2010). Lancet 376: 1417-1427.
9. Mitchell, JJ, Trakadis, YJ, and Scriver, CR (2011). Genet Med 13: 697-707.
10. van Spronsen, FJ (2010). Nat Rev Endocrinol 6: 509-514.
11. Kravtzoff, R et al. (1990). J Pharm Pharmacol 42: 473-476.
12. Kaufman, S (1987). Methods Enzymol 142: 3-17.
13. Nakata, H, Yamauchi, T, and Fujisawa, H (1979). J Biol Chem 254: 1829-1833.
14. Harding, CO, et al. (2004). Mol Genet Metab 81: 52-57.
15. Ohashi, A et al. (2012). Mol Genet Metab 105: 575-581.
16. Millan, CG et al., (2004). J Control Release 95: 27-49.
17. Updike, SJ (1985). Bibl Haematol: 65-74.
18. Kwon, YM, et al. (2009). J Control Release 139: 182-189.
19. Kravtzoff, R, et al. (1996). Eur J Clin Pharmacol 51: 221-225.
20. Kravtzoff, R, et al. (1996). Eur J Clin Pharmacol 49: 465-470.
21. Domenech, C, et al. (2011). Br J Haematol 153: 58-65.
22. Gamez A. et al., Molecular Therapy 2004, vol.9, No.1 : 124-129.
23. Sarkissian C. N. et al., Mol. Gen. And Metab. 86 (2005) S22-S26
24. Dufour, E, et al. (2012). Pancreas 41: 940-948.
25. Bourgeaux, V, et al. (2010). Transfusion 50: 2176-2184.

## Claims

1. A pharmaceutical composition comprising erythrocytes encapsulating phenylalanine hydroxylase (PAH) in a pharmaceutically acceptable carrier or vehicle,

2. The composition according to claim 1, wherein it is formulated in a dose volume comprising from 50 to 2000, preferably 100 to 1000 IU of PAH.

3. The composition according to claim 1 or 2, wherein it is formulated in a dose volume of 10 to 250 ml.

4. The composition according to any one of the preceding claims, wherein the haematocrit is between 40 and 70 %.

5. The composition according to any one of the preceding claims, comprising the cofactor BH₄.

6. The composition of any one of claims 1 to 5, for use in the treatment or prevention of phenylketonuria (PKU) in a patient.

7. The composition for the use according to claim 6, wherein the patient is submitted to a Phe-restricted diet.

8. The composition for the use according to claim 6 or 7, wherein the patient has a classic PKU.

9. The composition for the use according to claim 6 or 7, wherein the patient has a variant PKU and/or a non-PKU hyperphenylalaninemia.

10. The composition for the use according to any one of claims 6 to 9, wherein the patient is receiving the cofactor BH₄.

11. The composition for the use according to any one of claims 6 to 9, wherein the pharmaceutical composition is administered by intravenous or intra-arterial injection.

12. An erythrocyte encapsulating phenylalanine hydroxylase (PAH).

13. The erythrocyte of claim 12, in suspension in a pharmaceutically acceptable carrier or vehicle.

## Patentansprüche

1. Pharmazeutische Zusammensetzung, enthaltend Erythrozyten, die Phenylalanin-Hydroxylase (PAH) verkapseln, in einem pharmazeutisch annehmbaren Träger oder Mittel.

2. Zusammensetzung nach Anspruch 1, wobei sie in einem Dosisvolumen reichend von 50 bis 2000, vorzugsweise 100 bis 1000, IU von PAH formuliert ist.

3. Zusammensetzung nach Anspruch 1 oder 2, wobei sie in einem Dosisvolumen von 10 bis 250ml formuliert ist.

4. Zusammensetzung nach einem der vorstehenden Ansprüche, wobei der Hämatokrit zwischen 40 und 70 % liegt.

5. Zusammensetzung nach einem der vorstehenden Ansprüche, enthaltend den Cofaktor BH₄.

6. Zusammensetzung nach einem der Ansprüche 1 bis 5 zur Verwendung bei der Behandlung oder Prävention von Phenylketonurie (PKU) bei einem Patienten.

7. Zusammensetzung zur Verwendung nach Anspruch 6, wobei der Patient einer Phe-eingeschränkten Ernährung unterworfen ist.

8. Zusammensetzung zur Verwendung nach Anspruch 6 oder 7, wobei der Patient eine klassische PKU hat.

9. Zusammensetzung zur Verwendung nach Anspruch 6 oder 7, wobei der Patient eine PKU-Variante und/oder eine nicht-PKU Hyperphenylalaninämie hat.

10. Zusammensetzung zur Verwendung nach einem der Ansprüche 6 bis 9, wobei der Patient den Cofaktor BH₄ empfängt.

11. Zusammensetzung zur Verwendung nach einem der Ansprüche 6 bis 9, wobei die pharmazeutische Zusammensetzung durch intravenöse oder intraarterielle Injektion verabreicht wird.

12. Erythrozyt, der Phenylalaninhydroxylase (PAH) verkapselt.

13. Erythrozyt nach Anspruch 12, in Suspension in einem pharmazeutisch annehmbaren Träger oder Mittel.

## Revendications

1. Composition pharmaceutique comprenant des érythrocytes encapsulant de la phénylalanine hydroxylase (PAH) dans un support ou véhicule pharmaceutiquement acceptable.

2. Composition selon la revendication 1, qui est formulée dans un volume de dose comprenant 50 à 2000, de préférence 100 à 1000 Ul de PAH.

3. Composition selon la revendication 1 ou 2, qui est formulée dans un volume de dose de 10 à 250 ml.

4. Composition selon l'une quelconque des revendications précédentes, dans laquelle l'hématocrite est compris entre 40 et 70 %.

5. Composition selon l'une quelconque des revendications précédentes, comprenant le cofacteur BH₄.

6. Composition selon l'une quelconque des revendications 1 à 5, pour une utilisation dans le traitement ou la prévention d'une phénylcétonurie (PKU) chez un patient.

7. Composition pour une utilisation selon la revendication 6, dans laquelle le patient est soumis à un régime limité en Phe.

8. Composition pour une utilisation selon la revendication 6 ou 7, dans laquelle le patient a une PKU classique.

9. Composition pour une utilisation selon la revendication 6 ou 7, dans laquelle le patient a une variante de PKU et/ou une hyperphénylalaninémie non-PKU.

10. Composition pour une utilisation selon l'une quelconque des revendications 6 à 9, dans laquelle le patient reçoit le cofacteur BH₄.

11. Composition pour une utilisation selon l'une quelconque des revendications 6 à 9, dans laquelle la composition pharmaceutique est administrée par injection intraveineuse ou intra-artérielle.

12. Erythrocyte encapsulant de la phénylalanine hydroxylase (PAH).

13. Erythrocyte selon la revendication 12, en suspension dans un support ou véhicule pharmaceutiquement acceptable.
